# EUROPÄISCHE PATENTSCHRIFT

(11) **EP 2 121 922 B1**
(45) Veröffentlichungstag und Bekanntmachung des Hinweises auf die Patenterteilung: **12.01.2011**
(21) Anmeldenummer: 08706889.6
(22) Anmeldetag: 13.02.2008
(51) Int. Cl.: C12N 15/113, C07K 5/00, C07K 7/00

(54) **BIOLOGISCH WIRKSAME MOLEKÜLE, INSBESONDERE AUF GRUNDLAGE VON PNA UND SIRNA, VERFAHREN ZU DEREN ZELLSPEZIFISCHEN AKTIVIERUNG SOWIE APPLIKATIONSKIT ZUR VERABREICHUNG**
BIOLOGICALLY ACTIVE MOLECULES, PARTICULARLY BASED ON PNA AND SIRNA, METHOD FOR THE CELL-SPECIFIC ACTIVATION THEREOF, AND APPLICATION KIT TO BE ADMINISTERED
MOLÉCULES BIOLOGIQUEMENT ACTIVES, NOTAMMENT À BASE DE PNA ET DE SIRNA, PROCÉDÉ PERMETTANT LEUR ACTIVATION À SPÉCIFICITÉ CELLULAIRE, ET KIT D'APPLICATION POUR LEUR ADMINISTRATION

(30) Priorität: 15.02.2007 DE 102007008596
(43) Veröffentlichungstag der Anmeldung: 25.11.2009
(73) Patentinhaber: Friedrich-Schiller-Universität Jena, 07743 Jena (DE)
(72) Erfinder: PÖHLMANN, Tobias, 07743 Jena (DE); SEYFARTH, Lydia, 07751 Jena (DE)
(74) Vertreter: Donath, Dirk
(86) Internationale Anmeldenummer: PCT/DE2008/000279
(87) Internationale Veröffentlichungsnummer: WO 2008/098569

(56) Entgegenhaltungen:
- SCHIFFELERS RAYMOND M ET AL: "Cancer siRNA therapy by tumor selective delivery with ligand-targeted sterically stabilized nanoparticle" NUCLEIC ACIDS RESEARCH, OXFORD UNIVERSITY PRESS, SURREY, GB, Bd. 32, Nr. 19, 1. Januar 2004 (2004-01-01), Seite e149, XP002397466 ISSN: 0305-1048
- VORNLOCHER HANS-PETER: "Antibody-directed cell-type-specific delivery of siRNA." TRENDS IN MOLECULAR MEDICINE JAN 2006, Bd. 12, Nr. 1, Januar 2006 (2006-01), Seiten 1-3, XP002507095 ISSN: 1471-4914
- JIANG T ET AL: "Tumor imaging by means of proteolytic activation of cell-penetrating peptides" PROCEEDINGS OF THE NATIONAL ACADEMY OF SCIENCES OF USA, NATIONAL ACADEMY OF SCIENCE, WASHINGTON, DC.; US, Bd. 101, Nr. 51, 21. Dezember 2004 (2004-12-21), Seiten 17867-17872, XP002369426 ISSN: 0027-8424
- HATAKEYAMA H ET AL: "Development of a novel systemic gene delivery system for cancer therapy with a tumor-specific cleavable PEG-lipid." GENE THERAPY JAN 2007, Bd. 14, Nr. 1, Januar 2007 (2007-01), Seiten 68-77, XP002507096 ISSN: 0969-7128
- ZHANG YAN ET AL: "Protease-modulated cellular uptake of quantum dots." NANO LETTERS SEP 2006, Bd. 6, Nr. 9, September 2006 (2006-09), Seiten 1988-1992, XP002507097 ISSN: 1530-6984

## Beschreibung

Biologisch wirksame Moleküle, insbesondere auf Grundlage von PNA und siRNA, Verfahren zu deren zellspezifischen Aktivierung sowie Applikationskit zur Verabreichung

Die Erfindung betrifft spezielle biologisch wirksame Moleküle, insbesondere auf Grundlage von "peptide nucleic acids" (PNA) und "short interfering RNA" (siRNA), ein Verfahren um diese in eine Zielzelle zu transfizieren und in dieser Zielzelle, bzw. unmittelbar vor der Transfektion zellspezifisch zu aktivieren, sowie einen Applikationskit zur Verabreichung in Verbindung mit einem Transfektionssystem. Die besagten biologisch wirksamen Moleküle interagieren nach ihrer Aktivierung mit der mRNA des Zielgens und im Falle von siRNA bilden sie zusammen mit speziellen Endoribonukleasen einen RNA-Proteinkomplex mit der Bezeichnung "RISC" (RNA induced silencing complex). Der RISC Komplex bindet an die Target-mRNA, wobei Endonukleasen die Ziel-mRNA schneiden. Auf diese Weise wird die Genexpression verhindert und somit das Entstehen von Zielproteinen gehemmt. Im Falle der Verwendung von aktivierten PNA Molekülen wird mit der Bindung an die Ziel-mRNA die Translation verhindert.

Die zellspezifisch aktivierbaren, biologisch wirksamen Moleküle können beispielsweise zur Bekämpfung und Wachstumshemmung anormaler Zellen, insbesondere bei der Tumorbehandlung, bei der Behandlung von Virus-Infektionen, bei alterungsspezifischen Behandlungen etc. eingesetzt werden. Allgemein können die zellspezifisch aktivierbaren, biologisch wirksamen Moleküle zur Modulation der Genexpression der Zielzellen genutzt werden. Dabei kann die Expression von Genen nicht nur verringert, sondern auch erhöht werden, indem durch die biologisch aktiven Moleküle eine Verringerung der Expression der Negativ-Regulatoren des Zielgens erreicht wird.

Die Hemmung der Genexpression durch Einbringen von kurzen (19-23bp), doppelsträngigen RNA-Molekülen (siRNA) bzw. PNA Molekülen in eukaryotische Zellen, die spezifisch für einen Sequenzabschnitt der mRNA eines Zielgens ist, wurde bereits beschrieben (Elbashir SM et al.: Duplexes of 21-nucleotide RNAs mediate RNA interference in cultured mammalian cells, Nature, 2001 May 24, 411(6836), 494-8; Liu Y et al.: Efficient and isoform-selective inhibition of cellular gene expression by peptide nucleic acids, Biochemistry, 2004 Feb 24, 43(7), 1921-7; US 5,898,031; US 7,056,704).

Mit Hilfe solcher Moleküle wird nicht das Ablesen eines Gens und die Produktion einer mRNA verhindert, sondern es wird im Falle von siRNA ein zelleigener Mechanismus initiiert, der die Target-mRNA abbaut. Schließlich wird, wie vorbeschrieben, die Bildung eines spezifischen Proteins unterdrückt, ohne die Expression weiterer Gene zu beeinträchtigen (post-transcriptional gene silencing).

Um die Expression eines Gens zu unterdrücken, können die siRNA und PNA Moleküle dabei insbesondere über Transfektionsreagenzien und Elektroporation direkt in die Zelle eingebracht werden (Zhang M et al.: Downregulation enhanced green fluorescence protein gene expression by RNA interference in mammalian cells, RNA Biol. 2004 May, 1(1), 74-7; Gilmore IR et al.: Delivery strategies for siRNA-mediated gene silencing, Epub 2004 May 22., Curr Drug Deliv. 2006 Apr, 3(2), 147-5; US 6,506,559).

Von Nachteil ist dabei, dass die siRNA relativ instabil ist, was durch chemische Modifikationen verbessert werden kann (US 6,107,094).

Ein spezielles Problem der therapeutischen Anwendung von biologisch wirksamen Molekülen ist eine Applikation in vivo. Für eine solche Applikation wurden Möglichkeiten entwickelt, die siRNA zu stabilisieren, um den Abbau zu vermindern (Morrissey et. al.: "Chemical Modifications of Synthetic siRNA", Pharmaceutical Discovery, May 1, 2005), und es wurden Transfektionsreagenzen, beispielsweise Nanopartikel, in vivo-jetPEI™ (Polyplus), entwickelt, welche auch in vivo die siRNA in Zellen einbringen (Vernejoul et al.: Antitumor effect of in vivo somatostatin receptor subtype 2 gene transfer in primary and metastatic pancreatic cancer models, Cancer Research 62, 2002, 6124-31; Urban-Klein B, Werth S, Abuharbeid S, Czubayko F, Aigner A: RNAi-mediated gene-targeting through systemic application of polyethylenimine (PEI)-complexed siRNA in vivo, Gene Ther 12(5), 2005, 461-6.).

Ebenfalls wurden Methoden entwickelt, verstärkt Zellen eines Zielgewebes mit siRNA in vivo zu transfizieren (Ikeda et. al.: "Ligand-Targeted Delivery of Therapeutic siRNA", Pharmaceutical Research, Vol. 23, No. 8, August 2006).

Die Verabreichung von biologisch aktiven Substanzen in vivo ist allerdings auf Grund der systemischen Wirkung oft problematisch. Das Einbringen dieser Substanzen selektiv in Zielzellen erfolgt nicht ausreichend spezifisch. Dies ist besonders bei siRNA- und PNA-Molekülen von Nachteil, die selektiv und ausschließlich in Zielzellen zur Wirkung kommen sollen. Durch gewebs- bzw. zellspezifisch-markierte Transfektionsreagenzien (z. B. Antikörper/Antigen-markierte Nanopartikel, TAT-Protein-Flankierung, u. a.) wird keine genügend große Zellspezifität erreicht. Fehltransfektionen sind die Folge.

Weiterhin ist bekannt, siRNA-Moleküle durch Bindung von Fluorochromen in ihrer biologischen Wirkung zu inaktivieren und durch Bestrahlung mit Licht einer bestimmten Wellenlänge in ihre aktive Form wieder zu überführen (QN Nguyen et al.: Light controllable siRNAs regulate gene suppression and phenotypes in cells, Biochim Biophys Acta, 2006). Diese Aktivierung wird von außen initiiert und erfolgt in keiner Weise zellspezifisch. Dadurch wirken die besagten siRNA-Moleküle nach ihrer Aktivierung wiederum auch ungewollt in allen anderen transfizierten Zellen und nicht nur in den bestimmungsgemäßen Zielzellen.

Außerdem kann auch dieser Mechanismus nur schwer in vivo angewandt werden.

Die Publikation von Schiffelers Raymond M. et al: "Cancer siRNA therapy by tumor selctive delivery with ligand-targeted sterically stabilized nanoparticle" in Nucleic Acids Resarch, Oxfort University Press, Surry, GB, Bd. 32, Nr. 19, 1. Januar 2009, Seite 149 offenbart ein tumorspezifisches Transportreagenz bestehend aus PEI und ein Peptidligand mit RGD-Motif zum Einbringen der siRNA in Zielzellen. Die Publikation von Jiang T. et. Al: "Tumor imaging by means of proteolytic activation of cellpenetrating peptides" in Proceedings of the National Academy of Sciences of USA, National Academy of sience, Washington, DC.; US, Bd. 101, Nr. 51 , 21. Dezember 2004, Seiten 17867-17872 offenbart ähnliche Konstrukte zum Einbringen der Fluorophore in Zielzellen.

Der Erfindung liegt die Aufgabe zu Grunde, biologische Wirksubstanzen in Form von Molekülkonstrukten zu schaffen, welche sowohl in vitro als auch in vivo in eine Zielzelle transfizierbar sind und ausschließlich dort die Expression von Genen hemmen, ohne bei anderen Zellen des Organismus die wirkstoffspezifische Expression des Zielgens und damit die Bildung von Proteinen zu beeinflussen.

Tumorzellen selektiv zu hemmen, ohne gesunde Zellen, die auch von den Wirkstoffen erreicht werden können, sowie deren Fortbestand zu beeinträchtigen.

Erfindungsgemäß werden die biologisch wirksamen Moleküle, insbesondere PNA und siRNA, kovalent an ein oder mehrere Peptide gebunden, welche jeweils wenigstens eine zu zielzellentypischen Enzymen ausgewählte und für die kovalente Bindung sowie deren Aufbrechen jeweils signifikante Aminosäuresequenz aufweisen. Durch diese kovalente Bindung werden die biologisch wirksamen Moleküle inaktiviert. Es findet somit nach der Transfektion in Zellen keine Hemmung einer spezifischen Genexpression statt, solang auch nur eine der gebundenen Peptidketten durch das Nichtvorhandensein des entsprechenden zielzellentypischen Enzyms an den PNA- bzw. siRNA-Molekülen verbleibt.

Durch ein geeignetes Transfektionssystem, beispielsweise Nanopartikel oder Hüllmoleküle wie Liposomen, können die inaktivierten Wirkstoffmoleküle in die Zielzellen transfiziert werden. Dort werden die besagten inaktivierenden kovalenten Bindungen durch das oder die zellspezifischen Enzyme, welche für die Aminosäuresequenzen des oder der Kopplungspeptide relevant sind, zelltypisch aufgebrochen, wodurch das nunmehr in der Zielzelle befindliche und von Peptiden gelöste Molekül in seiner biologischen Wirksamkeit aktiviert wird. Dieses bindet darauf hin an die spezifische mRNA der Zielzelle und hemmt dadurch in an sich bekannter Weise die Genexpression in dieser speziellen Zelle.

In allen anderen Zellen des Organismus als die vorbestimmten Zielzellen, in welche die besagten inaktiven Molekülkonstrukte ebenfalls gelangen können, bleiben die Wirkstoffmoleküle inaktiv, da die kovalenten Bindungen zwischen dem biologisch wirksamen Molekül, insbesondere PNA und siRNA, und dem oder den Peptiden durch das Nichtvorhandensein des oder der zielzellenspezifischen Enzyme vollständig (keine Peptidbindung wurde aufgebrochen) oder teilweise (nicht alle Peptidbindungen wurden aufgebrochen) bestehen bleiben. Das biologisch wirksame Molekül geht auf Grund der weiterhin kovalenten Peptidbindung keine Bindung mit der mRNA dieser Zelle ein, bzw. es wird im Fall der Verwendung von siRNA kein RISC initiiert.

Wenngleich beispielsweise bei der Tumorbehandlung die zu transfizierenden erfindungsgemäßen Molekülkonstrukte in ihrer inaktiven (gebundenen) Form nicht nur in bzw. an tumorerkrankte Zielzellen gelangen, sondern (was in der Praxis kaum zu vermeiden ist) auch gesunde Zellen erreichen können, werden selektiv ausschließlich in oder an den tumorerkrankten Zielzellen mit dem dortigen zellspezifischen Enzymen das besagte Molekül in seiner biologischen Wirkung aktiviert und die wirkstoffbezogene Expression des Zielgens gehemmt. Diese Genexpression und damit die Proteinbildung für den Fortbestand der gesunden Zellen bleibt trotz der in diesen nicht erkrankten (bzw. für die beabsichtigte biologische Wirkung nicht zweckbestimmten) Zellen befindlichen, jedoch permanent inaktiven Molekülkonstrukte von diesem Wirkstoff unberührt.

Es ist auch möglich, eine oder mehrere besagte kovalente Bindungen bereits unmittelbar vor der Transfektion in die Zielzelle durch ein oder mehrere auf deren Oberfläche oder in deren Umgebung vorhandene zellspezifische Enzyme teilweise oder vollständig (bezogen auf die Gesamtzahl der kovalenten Peptidbindungen) zu aktivieren. Beispielsweise könnten mehrere und jeweils auf unterschiedliche Enzyme der Zielzelle ausgerichtete Peptidketten mit Aminosäuresequenzen vorhanden sein, von denen eine oder mehrere Bindungen bereits unmittelbar an der Oberfläche der Zielzelle oder in deren Umgebung aufgebrochen werden.

Es wäre auch denkbar, dass die biologisch wirksamen Moleküle im durch die besagten Peptidbindungen inaktiven Zustand bis zu den Zielzellen bzw. bis zum Zielgewebe gelangen und dort, insbesondere, wenn sie in diesem Bereich des Organismus keine anderen Zellen erreichen können, vollständig vor ihrer Transfektion in die Zielzelle aktiviert werden.

Durch die vorgeschlagene hochselektive Wirkung der Moleküle infolge Zielzellen-enzymspezifischer Inaktivität/Aktivierung können, die zu transfizierenden biologisch inaktiven Molekülkonstrukte bei entsprechender Peptid-Bindung (mit definierten Aminosäurekombinationen) systemisch verabreicht werden.

Die Molekülkonstrukte können zum besseren Transport in bzw. an die Zielzellen sowie zu ihrer Stabilisierung außerdem an weitere Stoffe (beispielsweise Nanopartikel als Trägersystem) gebunden werden.

Darüber hinaus können zur Verbesserung der Transfektionsrate an sich bekannte Wirkungsmechanismen, welche die Gewebs- oder Zellselektivität erhöhen (beispielsweise Ligand/Antikörper/Antigenmarkierte Nanopartikel, TAT-Protein-Flankierung) genutzt werden. Die Kombination mit diesen bekannten Wirkungsmechanismen ergänzen die beschriebene Erfindung und führen aufgrund der Vorselektion der Zielzellen zu einer Verringerung der einzusetzenden erfindungsgemäßen Wirkstoffmenge. Dies resultiert daraus, dass bei der Wirkstoffeinbringung in vivo in den Blutkreislauf vor allem Zellen in gut durchbluteten Geweben erreicht werden. Die vorgeschlagenen Moleküle mit der erfindungsgemäßen biologisch inaktivierenden Bindung an ein oder mehrere Peptide können sehr gut im Handling mit an sich bekannten Transfektionssystemen, wie beispielsweise Nanopartikel mit oder ohne Ligand/Antikörper/Antigenmarkierung, mit umhüllenden Nanopartikel oder Lipide sowie Lipid-basierte Transfektionsmethoden, angewendet werden. Solche Transfektionssysteme verringern je nach bestimmungsgemäßem Einsatz schon an sich organismusbedingte Fehltransfektionen, wodurch, in Relation gesehen, vermehrt Zellen im Zielgewebe transfiziert und auf diese Weise die zur Anwendung kommenden Wirkstoffmengen jeweils auf ein Minimum beschränkt werden können.

Durch Anwendung der Erfindung können diese Fehltransfektionen zwar nicht verhindert werden, jedoch sind die fehltransfizierten Moleküle in diesen anderen Zellen als die Zielzellen, wenngleich nach wie vor unerwünscht, biologisch nicht wirksam. Dieser Status bleibt auch trotz erfindungsgemäßer Molekülaktivierung in oder an den Zielzellen unverändert, so dass die biologische Wirkung selektiv ausschließlich in den Zielzellen erfolgt und im Gegensatz zu den bekannten Mechanismen eine hochgradig zellselektive Modulation der Genexpression erreicht wird.

Die Erfindung ist nicht auf Anwendungen zur Behandlung von Tumoren und beispielsweise Virus (z. B. HIV) infizierten Zellen beschränkt. Die erfindungsgemäßen Molekülkonstrukte können bei allen Krankheitsbildern, denen eine verstärkte oder verminderte Genexpression zu Grunde liegt, verwendet werden, um die Krankheit selbst oder deren Symptome zu behandeln. Weiterhin können alters-oder entwicklungsbedingte Veränderungen im Genexpressionsmuster von Zellen behandelt werden. Außerdem kann die Erfindung für alle Zwecke benutzt werden, deren Ziel die selektive Veränderung der Expression von Genen in bestimmten Zellen in vitro oder in vivo ist.

Vorteilhaft ist ein Applikationskit, mit welchem die einzusetzenden biologisch wirksamen Moleküle mit den gebundenen Peptiden, die vorschlagsgemäß in Hinsicht auf die besonderen Enzyme der Zielzellen ausgewählte Aminosäurensequenzen aufweisen, sowie ein jeweils geeignetes Transfektionssystem und sonstige Zusatzstoffe bereitgestellt werden. Der Applikationskit enthält alle notwendigen Inhaltsstoffe sowie eine Anwendungsanleitung für den bestimmungsgemäßen Einsatz durch einen Anwender.

Die Erfindung soll nachstehend anhand von in der Zeichnung dargestellten Ausführungsbeispielen näher erläutert werden.

Es zeigen:
- Fig. 1:: allgemeine Struktur der erfindungsgemäßen zellspezifisch aktivierbaren Molekülkonstrukte (inaktivierter Zustand; durch veränderte Raumstruktur der Moleküle wird im Beispiel a) die Formierung des RISC verhindert und im Beispiel b) die Bindung an die mRNA unterbunden) a) Beispiel für siRNA mit zwei gebundenen Peptiden b) Beispiel für PNA mit einem gebundenem Peptid
- Fig. 2:: Beispiel für ein siRNA Molekülkonstrukt, gebunden an ein Transfektionssystem zum Zweck der Transfektion in Zielzellen, Aktivierung dieses siRNA Molekülkonstrukts
a) biologisch inaktives Molekülkonstrukt zur Transfektion in die Zielzelle
b) Bindungsaufbruch durch das zielzellenspezifische Enzym
c) in oder an der Zielzelle biologisch aktiviertes Molekül mit aufgebrochener Bindung zwischen der siRNA und dem Peptid
- Fig. 3:: siRNA Molekülkonstrukt mit zwei Peptidbindungen und einem Transfektionssystem zur Transfektion in Zielzellen
a) Molekülkonstrukt mit Peptidbindungen
b) Aufbruch der Peptidbindungen durch unterschiedliche Enzyme der Zielzelle
- Fig. 4:: Darstellung einer beispielhaften Verbindung zwischen einem Peptid und einer siRNA, ein mögliches Enzym zur Spaltung der Peptidbindung ist in diesem Fall Caspase-4

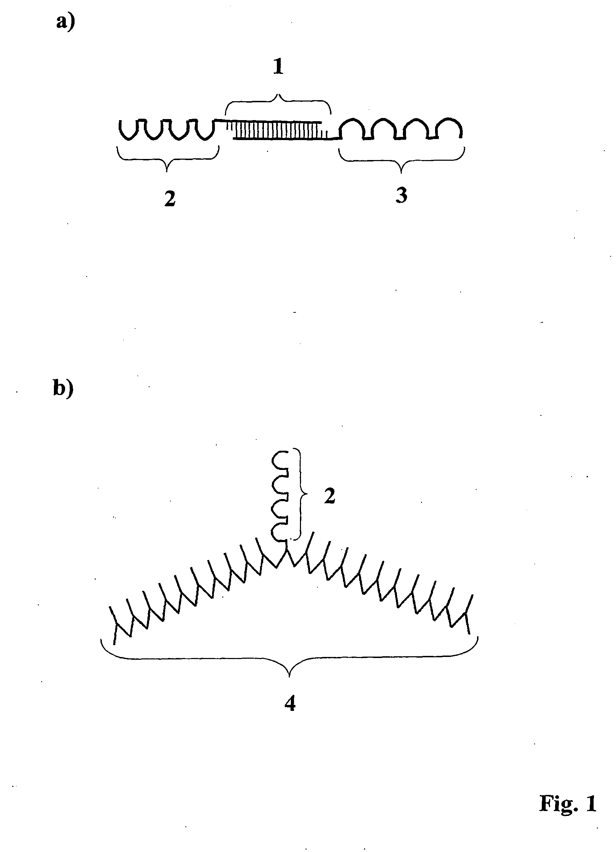

In Fig. 1 ist die allgemeine Struktur der erfindungsgemäßen zellspezifisch aktivierbaren Molekülkonstrukte im inaktivierten Zustand beispielhaft für siRNA (Fig. 1a) und für PNA (Fig. 1b) dargestellt.

In Fig. 1a ist eine siRNA 1 als biologisch wirksames Molekül an zwei Peptide 2, 3 gebunden. Mit diesen beidseitigen Peptidbindungen ist die siRNA 1 biologisch inaktiv und wird in eine (aus Übersichtsgründen nicht dargestellte) Zielzelle transfiziert.

Fig. 1b veranschaulicht, wie statt der siRNA 1 eine PNA 4 als biologisch wirksames Molekül an das Peptid 2 gebunden und damit zur Transfektion ebenfalls biologisch inaktiviert ist.

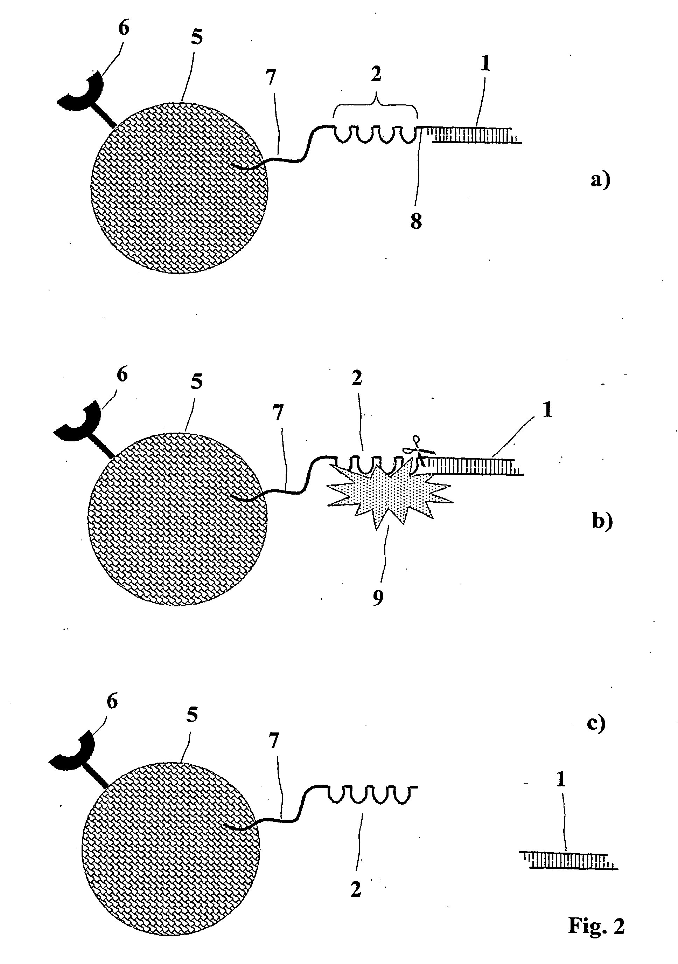

In Fig. 2 ist ein mögliches Konstrukt dargestellt, welches zur Transfektion des durch Peptidbindung (Peptid 2) inaktiven biologisch wirksamen Moleküls (siRNA 1) in eine Zielzelle zum Einsatz kommen könnte. Dabei könnten (Fig. 2a) an einem Nanopartikel 5 Antikörper 6 zur semi-selektiven Bindung an Zielzellen, sowie Polyethylenglycolketten (PEG) 7 zur Verankerung des Peptids 2 und der siRNA 1 gebunden sein.

Weiterhin ist die Bindung zwischen der siRNA 1 und dem inaktivierenden Peptid 2 als Schnittstelle 8 zum Aufbrechen durch ein selektiv schneidendes und zielzellenspezifisches Enzym 9 dargestellt (Fig. 2b). Dieses Enzym 9, welches nur in oder an der besagten (nicht dargestellten) Zielzelle vorhanden ist, bricht die Peptidbindung der siRNA 1 aufgrund der speziellen Aminosäuresequenz des Peptids 2 an der Schnittstelle 8 (Fig. 2c) auf. Das durch die aufgebrochene Peptidbindung nunmehr wieder aktive biologisch wirksame Molekül (siRNA 1) sowie das restliche Konstrukt, bestehend aus dem Nanopartikel 5, dem Antikörper 6, den Polyethylenglycolketten (PEG) 7 und dem von der siRNA 1 gelösten Peptid 2, sind somit separiert.

In oder an anderen Zellen des Organismus als den bestimmungsgemäßen Zielzellen, in welche das biologisch inaktive Molekülkonstrukt (siehe Fig. 2a und 2b) durch Transfektion ebenfalls gelangt und in oder an denen jedoch das zielzellenspezifische Enzym 9 nicht vorhanden ist, bleibt die Peptidbindung der siRNA 1 an der Schnittstelle 8 erfindungsgemäß erhalten. Die siRNA 1 ist als biologisch wirksames Molekül weiterhin inaktiv (vgl. Fig. 2a). Die in den Zielzellen bezweckte biologische Wirkung der siRNA 1 wird durch die nicht aufgebrochene Schnittstelle 8 in anderen Zellen nicht ausgeführt.

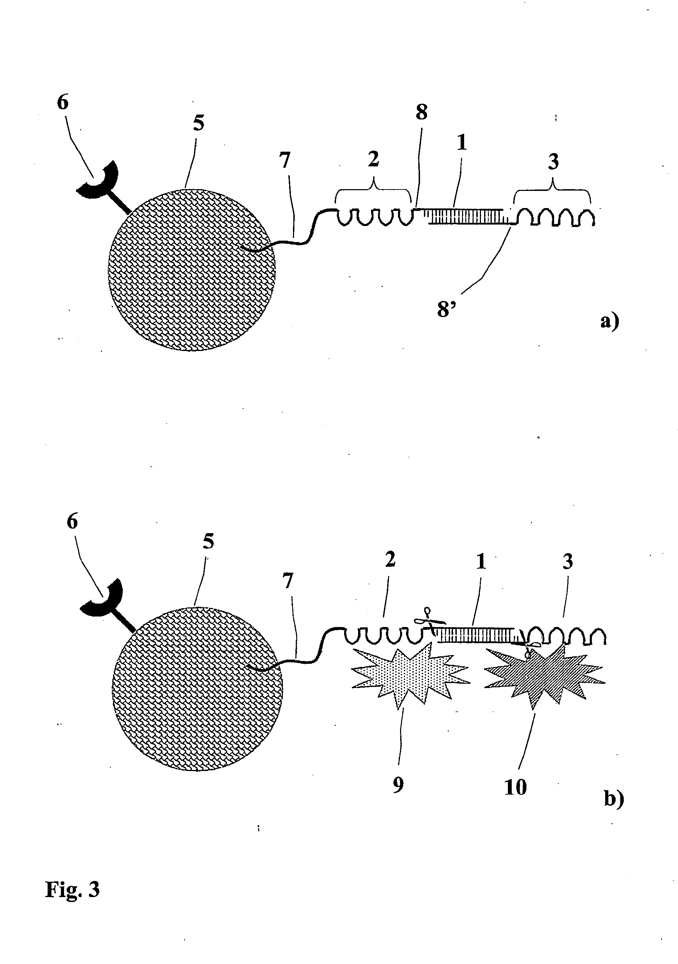

In Fig. 3a ist eine Erweiterung des Konstrukts aus Fig. 2 dargestellt. Dabei ist wiederum zur biologischen Inaktivierung der siRNA 1 an verschiedenen Stellen derselben jeweils ein Peptid 2, 3 gebunden (vgl. Fig. 1a). Die verschiedenen Aminosäuresequenzen in den gebundenen Peptiden 2, 3 sind so gewählt, dass zwei (ausschließlich) in oder an der Zielzelle vorhandene spezifische Enzyme 9, 10 die Peptidbindungen dieser Schnittstellen 8 bzw. 8' jeweils aufbrechen können. Sollte auch nur eines der beiden Peptide 2, 3 bei einer anderen Zelle als der besagten Zielzelle, in bzw. an das Konstrukt gemäß Fig. 3a (z. B. nach Fehltransfektion) ebenfalls gelangt, nicht vorhanden sein, bleibt zumindest einer der beiden Peptidbindungen an den Schnittstellen 8 bzw. 8' auf Grund des Fehlens des äquivalenten zielzellenspezifischen Enzyms 9, 10 erhalten. Die siRNA 1 wäre selbst bei einer einzigen noch bestehenden Peptidbindung weiterhin inaktiv. Ausschließlich bei der Zielzelle, in oder an welcher die Enzyme 9, 10 durch die vorgenannten definierten Aminosäuresequenzen der Peptide 2, 3 beide Schnittstellen 8, 8' aufbrechen (angedeutet in Fig. 3b), wird die siRNA 1 vom übrigen Molekülkonstrukt vereinzelt (vgl. auch Fig. 2c). Somit kann die siRNA 1 als biologisch aktives Molekül auch nur in dieser Zielzelle die mit der Transfektion bezweckte Wirkung entfalten.

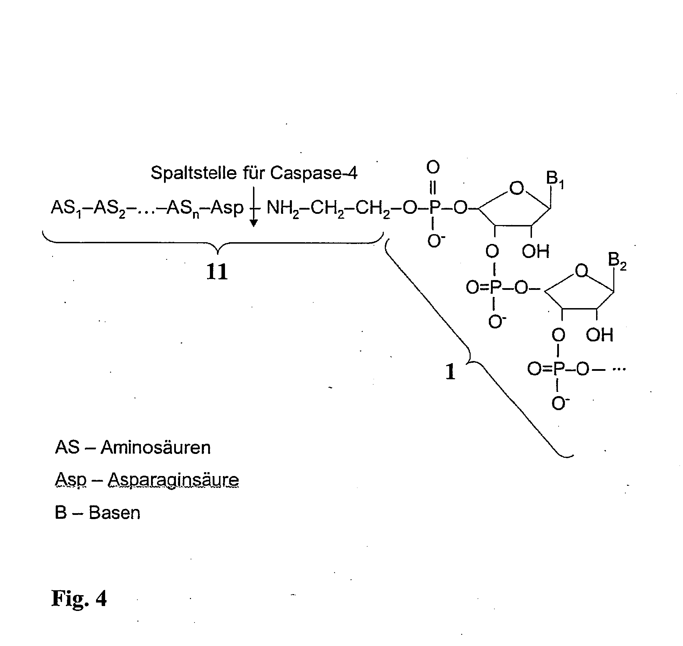

Fig. 4 zeigt eine mögliche Verbindung zwischen der siRNA 1 und einem Peptid mit einer möglichen spezifischen Aminosäuresequenz 11 (Aminosäuresequenz ist -L-E-V-D-) für ein in einer Zielzelle vorhandenes selektiv spaltendes Enzym Caspase-4 dargestellt. Dieses würde die Verbindung zur Aktivierung der siRNA 1 an der durch Pfeildarstellung symbolisierten und bezeichneten Spaltstelle für Caspase-4 aufbrechen.

Im abgebildeten Bespiel würde nach besagter Abspaltung durch das Enzym Caspase-4 ein Molekülrest an der siRNA 1 verbleiben, was aber deren biologische Aktivität nicht beeinträchtigt.

Die Erfindung ist nicht auf die vorgenannte und in Fig. 4 dargestellte Aminosäuresequenz (-L-E-V-D-) für den Aufbrauch der Peptidbindung an der siRNA 1 durch das zielzellenspezifische Enzym Caspase-4 beschränkt. In nachstehender Tabelle sind weitere Aminosäuresequenzen des Peptids zur vorschlagsgemäßen Verwendung für spezielle Zielzellenenzyme beispielhaft zusammengestellt:

| **Zielzellenenzym** | **Aminosäuresequenz des Peptids** |
|---|---|
| Matrix Metalloproteinase-1 | -Pro-Leu-Ala-Leu-Trp-Ala-Arg- |
| Matrix Metalloproteinasen-2,7 | -Pro-Leu-Gly-Leu-Dpa-Ala-Arg- |
| Matrix Metalloproteinasen-2,9 | -Pro-Leu-Gly-Met-Trp-Ser-Arg- |
| Matrix Metalloproteinasen-3,1,2,9 | -Arg-Pro-Lys-Pro-Tyr-Ala-Nval-Trp-Met-Lys- |
| Cathepsin-S | -Phe-Arg-Phe(p-nitro)- |
| Cathepsin-G | -Ala-Ala-Phe- |
| Cathepsin-D | -Arg-Gly-Phe-Phe-Leu- |
| Angiotensin-Converting-Enzym | -Gly-His-Leu- oder -Phe-Gly-Gly- oder -Gly-p-Nitro-Phe-Pro- |

Die vier in vorstehender Tabelle erstgenannten Zielzellenenzyme können auch auf der Oberfläche der Zielzelle bzw. in deren Umgebung vorkommen und wären in diesem Fall in der Lage, für eine solche Anwendung der biologisch wirksamen Moleküle die entsprechende Peptidbindung mit der erfindungsgemäß ausgewählten Aminosäuresequenz bereits unmittelbar vor der Transfektion in die Zielzelle aufzubrechen. Diese Verwendung wäre insbesondere denkbar, wenn die im inaktiven Zustand in den Bereich der Zielzelle gelangten biologisch wirksamen Moleküle nach ihrer gänzlich oder teilweise bereits außerhalb der Zielzelle erfolgten Aktivierung in diesem Teil des Organismus keine anderen (unerwünschten) Zellen mehr erreichen können, beispielsweise zur Transfektion in einen Zielzellenkomplex.

Vorteilhaft ist ein Applikationskit, in welchem die einzusetzenden biologisch wirksamen Moleküle mit den gebundenen Peptiden, die vorschlagsgemäß in Hinsicht auf die besonderen Enzyme der Zielzellen ausgewählte Aminosäurensequenzen aufweisen, bereitgestellt werden. Der Applikationskit sollte in Ampullenform alle notwendigen Inhaltsstoffe, in zweckmäßiger Weise auch eine Auswahl geeigneter Transfektionssysteme (wie Nanopartikel oder Lipide), sonstige Zusatzstoffe (wie Antikörper, Liganden und Polyethylenglycol) sowie eine oder mehrere Sonden bzw. Spritzen mit Kanüle zur Injektion der Mischung aus den Ampulleninhalten in das die Zielzellen aufweisende Medium enthalten. Gemäß einer beiliegenden Anwendungsanleitung mit einer Gegenüberstellung der auswählbaren Peptid-Aminosequenzen zu jeweiligen Enzymen der Zielzellen (vgl. vorstehende Tabelle) kann der Nutzer für den bestimmungsgemäßen Einsatz entsprechende Applikationsmischungen zusammenstellen und zur Anwendung bringen.

Es bietet sich an, einen solcher Applikationskit spezifisch für ausgewählte Zielzellen und Zielgene sowie je nach Anwendungsbereich (in vitro oder in vivo) bereitzustellen.

**Bezugszeichenliste**

| | | |
|---|---|---|
| 1 | - | siRNA |
| 2, 3 | - | Peptid |
| 4 | - | PNA |
| 5 | - | Nanopartikel |
| 6 | - | Antikörper, Ligand |
| 7 | - | Polyethylenglycol (PEG) |
| 8, 8' | - | Schnittstelle (Bindung) zwischen Peptid und siRNA |
| 9, 10 | - | Enzym der Zielzelle, welches die Verbindung zwischen Peptid und siRNA zu dessen Aktivierung spaltet |
| 11 | - | Aminosäuresequenz des Peptids (im Beispiel: spezifische Aminosäuresequenz für das Enzym Caspase-4) |

### Sequenzprotokoll

### ALLGEMEINE ANGABEN:

### ANMELDER:

NAME: Friedrich-Schiller-Universität Jena
STRASSE: Fürstengraben 1
ORT: Jena
BUNDESLAND: Thüringen
LAND: Deutschland
POSTLEITZAHL: 07743
TELEFON: 03641-931074
TELEFAX: 03641-931072

### BEZEICHNUNG DER ERFINDUNG:

Biologisch wirksame Moleküle, insbesondere auf Grundlage von PNA und siRNA, Verfahren zu deren zellspezifischen Aktivierung sowie Applikationskit zur Verabreichung

### ANZAHL DER SEQUENZEN: 11

### COMPUTERLESBARE FASSUNG:

DATENTRÄGER: Diskette
COMPUTER:, PC
BETRIEBSSYSTEM: Microsoft Windows 2000
SOFTWARE: Microsoft Word.

### DATEN DER JETZIGEN ANMELDUNG:

DE 10 2007 008 596.8

### ANGABEN DER SE ID-NO: 1 BIS ID-NO: 11

### SEQUENZCHARAKTERISTIKA:

ART: Aminosäuren
TOPOLOGIE: linear

### ART DES MOLEKÜLS: Oligopeptid

SEQ ID-NO: 1
   -Leu-Glu-Val-Asp- 4
SEQ ID-NO: 2
   -Pro-Leu-Ala-Leu-Trp-Ala-Arg- 7
SEQ ID-NO: 3
   -Pro-Leu-Gly-Leu-Dpa-Ala-Arg- 7
SEQ ID-NO: 4
   -Pro-Leu-Gly-Met-Trp-Ser-Arg- 7
SEQ ID-NO: 5
   -Arg-Pro-Lys-Pro-Tyr-Ala-Nval-Trp-Met-Lys- 10
SEQ ID-NO: 6
   -Phe-Arg-Phe(p-nitro)- 3
SEQ ID-NO: 7
   -Ala-Ala-Phe- 3
SEQ ID-NO: 8
   -Arg-Gly-Phe-Phe-Leu- 5
SEQ ID-NO: 9
   -Gly-His-Leu- 3
SEQ ID-NO: 10
   -Phe-Gly-Gly- 3
SEQ ID-NO: 11
   -Gly-p-Nitro-Phe-Pro- 4

## Patentansprüche

1. PNA- oder siRNA- Moleküle zur biologisch inaktiven Transfektion in eine Zielzelle, um in dieser nach biologischer Aktivierung durch Bindung an mRNA und beispielsweise im Fall von siRNA unter Formierung eines "RISC"-Komplexes die Expression von Genen zu hemmen, **dadurch gekennzeichnet, dass** die PNA- oder siRNA-Moleküle (1) zum Zweck ihrer biologischen Inaktivierung außerhalb der Zielzelle zumindest eine nur durch das oder die zellspezifischen Enzyme (9, 10) dieser Zielzelle aufbrechbare kovalente Bindung (8, 8') mit wenigstens einem Peptid (2, 3) besitzen, welches jeweils eine oder mehrere zu dem oder den zielzellentypischen Enzymen ausgewählte und für die aufbrechbare kovalente Bindung (8, 8') jeweils signifikante Aminosäuresequenzen (11) aufweist.

2. PNA- oder siRNA- Moleküle nach Anspruch 1, **dadurch gekennzeichnet, dass** das wenigstens eine Peptid (2, 3) zum Zweck des Aufbrechens der Bindung (8, 8') durch das zellspezifische Enzym Caspase-4 die Aminosäuresequenz -L-E-V-D- oder Matrix Metalloproteinase-1 die Aminosäuresequenz -Pro-Leu-Ala-Leu-Trp-Ala-Arg- oder Matrix Metalloproteinasen-2,7 die Aminosäuresequenz -Pro-Leu-Gly-Leu-Dpa-Ala-Arg- oder Matrix Metalloproteinasen-2,9 die Aminosäuresequenz -Pro-Leu-Gly-Met-Trp-Ser-Arg- oder Matrix Metalloproteinasen-3,1,2,9 die Aminosäuresequenz -Arg-Pro-Lys-Pro-Tyr-Ala-Nval-Trp-Met-Lys- oder Cathepsin-S die Aminosäuresequenz -Phe-Arg-Phe(p-nitro)- oder Cathepsin-G die Aminosäuresequenz -Ala-Ala-Phe- oder Cathepsin-D die Aminosäuresequenz -Arg-Gly-Phe-Phe-Leu- oder Angiotensin-Converting-Enzym die Aminosäuresequenz -Gly-His-Leu- oder Angiotensin-Converting-Enzym die Aminosäuresequenz -Phe-Gly-Gly- oder Angiotensin-Converting-Enzym die Aminosäuresequenz - Gly-p-Nitro-Phe-Pro- aufweist.

3. Verfahren zur zellspezifischen Aktivierung von PNA- oder siRNA-Molekülen in oder an einer Zielzelle, bei dem die PNA- oder siRNA-Moleküle vorzugsweise biologisch inaktiv in eine Zielzelle transfiziert werden, um dort nach deren biologischer Aktivierung unter Bindung an mRNA und beispielsweise im Fall von siRNA unter Formierung eines "RISC"-Komplexes die Expression von Genen zu hemmen, **dadurch gekennzeichnet, dass** das PNA- oder siRNA-Molekül zum Zweck dessen biologischer Inaktivierung außerhalb der Zielzelle bzw. vor der Transfektion kovalent an ein oder mehrere Peptide mit jeweils zumindest einer zu dem oder den zielzellentypischen Enzymen ausgewählten und für die aufbrechbare kovalente Bindung signifikante Aminosäuresequenz gebunden wird und dass die kovalente Bindung des oder der Peptide an das PNA-oder siRNA- Molekül zum Zweck dessen biologischer Aktivierung jeweils durch das zielzellentypische Enzym aufgebrochen wird.

4. Verfahren nach Anspruch 3, **dadurch gekennzeichnet, dass** das PNA-oder siRNA- Molekül mit dem oder den kovalent gebundenen Peptiden zur Verbesserung der Transfektion in Zielzellen an ein Transfektionssystem gekoppelt wird.

5. Verfahren nach Anspruch 4, **dadurch gekennzeichnet, dass** als Transfektionssystem ein Trägersystem, beispielsweise Nanopartikel mit oder ohne Ligand/Antikörper/Antigenmarkierung, zum Einsatz kommt.

6. Verfahren nach Anspruch 4, **dadurch gekennzeichnet, dass** als Transfektionssystem Moleküle, beispielsweise Nanopartikel oder Lipide, dienen, mit denen die selektiv aktivierbaren PNA- oder siRNA- Moleküle zu ihrer Transfektion umhüllt werden.

7. Verfahren nach Anspruch 4, **dadurch gekennzeichnet, dass** als Transfektionssystem Lipid-basierte Methoden Verwendung finden.

8. Verfahren nach Anspruch 4, **dadurch gekennzeichnet, dass** als Transfektionssystem eine TAT-Protein-Flankierung vorgesehen ist.

9. Verfahren nach Anspruch 3, **dadurch gekennzeichnet, dass** das PNA-oder siRNA- Molekül im inaktivierten Zustand in die Zielzelle transfiziert und in dieser durch das oder die zellspezifischen Enzyme aktiviert wird.

10. Verfahren nach Anspruch 3, **dadurch gekennzeichnet, dass** das durch Peptidbindung inaktivierte PNA- oder siRNA- Molekül bereits unmittelbar vor der Transfektion in die Zielzelle durch ein oder mehrere auf deren Oberfläche oder in deren Umgebung vorhandene zellspezifische Enzyme (z. B. Matrix Metalloproteinase-1, Matrix Metalloproteinasen-2, Matrix Metalloproteinasen-3, Matrix Metalloproteinasen-7, Matrix Metalloproteinasen-9) teilweise oder vollständig (bezogen auf die Gesamtzahl der kovalenten Peptidbindungen) aktiviert wird.

11. Verfahren nach Anspruch 3, **dadurch gekennzeichnet, dass** das PNA-oder siRNA- Moleküle mit dem oder den kovalent gebundenen Peptiden an ein oder mehrere Markersubstanzen gekoppelt wird.

12. Applikationskit zur Verabreichung der PNA- oder siRNA- Moleküle gemäß einem oder mehreren der Ansprüche 1 bis 11, bestehend zumindest aus
- wenigstens einer Ampulle (Ampulle A), welche eine, zum Zweck der Inaktivierung an ein ausgewähltes Peptid mit einer speziellen Aminosäuresequenz (beispielsweise die Aminosäuresequenz -Pro-Leu-Ala-Leu-Trp-Ala-Arg- für das Zielzellenenzym Matrix Metalloproteinase-1) gebundene, siRNA enthält und/oder wenigstens einer Ampulle (Ampulle B), welche eine, zum Zweck der Inaktivierung an ein ausgewähltes Peptid mit einer speziellen Aminosäuresequenz (beispielsweise die Aminosäuresequenz -Pro-Leu-Ala-Leu-Trp-Ala-Arg- für das Zielzellenenzym Matrix Metalloproteinase-1)gebundene, PNA enthält,
- wenigstens einer weiteren Ampulle (Ampulle C) mit einem Transfektionssystem, beispielsweise Nanopartikel oder Lipide,
- Verdünnungs- und Reaktionspuffer für die Inhalte der Ampullen A bis C,
- einer oder mehreren Sonden bzw. Spritzen mit Kanüle und andere benötigte Materialien zur Injektion der Mischung aus den Ampulleninhalten in das die Zielzellen enthaltende Medium sowie
- einer Anleitung zur Verabreichung mit einer Gegenüberstellung der auswählbaren Peptid-Aminosequenzen für jeweilige Enzyme der Zielzelle.

13. Applikationskit gemäß Anspruch 12, enthaltend wenigstens eine Substanz, beispielsweise Antikörper zur semi-selektiven Bindung der PNA- oder siRNA- Moleküle an Zielzellen.

14. Applikationskit gemäß Anspruch 12, enthaltend wenigstens eine Substanz, beispielsweise Polyethylenglycolketten, zur Verankerung des Peptids und der PNA- oder siRNA- Moleküle.

15. Applikationskit gemäß Anspruch 12, enthaltend wenigstens eine Substanz, beispielsweise Lipid-basierendes Transfektionsreagenz, zur Transfektion der PNA- oder siRNA- Moleküle in die Zielzelle.

16. Applikationskit gemäß Anspruch 12, **dadurch gekennzeichnet, dass** dieser für in vitro- oder vivo-Anwendungen bereitgestellt wird.

## Claims

1. PNA or siRNA molecules for the biologically inactive transfection into a target cell to inhibit the gene expression therein after biological activation by bonding to mRNA and, for example, in the case of siRNA under the formation of a RISC complex, wherein for their biological deactivation outside the target cell the PNA or siRNA molecules (1) have at least one covalent bond (8, 8') that can only be broken up by the one or more cell-specific enzymes (9, 10) of said target cell, and said covalent bond has at least one peptide (2, 3) that has always one or more amino acid sequences (11) that is/are selected for the one or more enzymes typical for the target cell and is/are significant for the breakable covalent bond (8, 8').

2. PNA or siRNA molecules according to claim 1, wherein the at least one peptide (2, 3) contains the amino acid sequence -L-E-V-D- or matrix metalloproteinase-1, the amino acid sequence -Pro-Leu-Ala-Leu-Trp-Ala-Arg- or matrix metalloproteinases-2,7, amino acid sequence -Pro-Leu-Gly-Leu-Dpa-Ala-Arg- or matrix metalloproteinases-2,9, the amino acid sequence -Pro-Leu-Gly-Met-Trp-Ser-Arg- or matrix metalloproteinases-3,1,2,9, the amino acid sequence -Arg-Pro-Lys-Pro-Tyr-Ala-Nval-Trp-Met-Lys- or cathepsin-S, the amino acid sequence -Phe-Arg-Phe(p-nitro)- or cathepsin-G, the amino acid sequence -Ala-Ala-Phe- or cathepsin-D, the amino acid sequence -Arg-Gly-Phe-Phe-Leu- or angiotensin-converting enzyme, the amino acid sequence -Gly-His-Leu- or angiotensin-converting enzyme, the amino acid sequence -Phe-Gly-Gly- or angiotensin-converting enzyme, the amino acid sequence - Gly-p-Nitro-Phe-Pro--Pro-Leu-Ala-Leu-Trp-Ala-Arg- for breaking up the bond (8, 8') by the cell-specific enzyme caspase-4.

3. Method for the cell-specific activation of PNA and siRNA molecules in or at a target cell in which the PNA or siRNA molecules are transfected in their preferably biologically deactivated state into a target cell to inhibit the gene expression therein after their biological activation by bonding to mRNA and, for example, in the case of siRNA under the formation of a RISC complex, wherein the PNA or siRNA molecule for its biological deactivation outside the target cell or before the transfection is covalently bonded to one or more peptides each having at least one amino acid sequence that is selected for the one or more enzymes typical for the target cell and is significant for the bond to be broken up, and the covalent bond of the one or several peptides to the PNA or siRNA molecule is broken up by the enzymes typical of the target cell in order to activate the biological effect of said molecule.

4. Method according to claim 3, wherein the PNA or siRNA molecule with one or more covalently bonded peptides is coupled to a transfection system for improving the transfection in target cells.

5. Method according to claim 4, wherein a carrier system; for example nanoparticles with or without ligand/antibody/anti gene marking, is used as the transfection system.

6. Method according to claim 4, wherein molecules, for example nanoparticles or lipids, are used as the transfection system and envelope the PNA or siRNA molecules, which can be selectively activated, for their transfection.

7. Method according to claim 4, wherein lipid-based methods are used as the transfection system.

8. Method according to claim 4, wherein a TAT protein flanking is used as the transfection system.

9. Method according to claim 3, wherein the PNA or siRNA molecule is transfected in its deactivated state into the target cell and is activated by the one or more cell-specific enzymes therein.

10. Method according to claim 3, wherein before its transfection into the target cell the PNA or siRNA molecule deactivated by the peptide bond is already partly or completely (with respect to the total number of the covalent peptide bonds) activated by one or more cell-specific enzymes (e.g. matrix metalloproteinase-1, matrix metalloproteinases-2, matrix metalloproteinases-3, matrix metalloproteinases-7, matrix metalloproteinases-9) being present on the surface or vicinity of the target cell.

11. Method according to claim 3, wherein the PNA or siRNA molecule with the one or more covalently bonded peptides is coupled to one or more marker substances.

12. Application kit for administering PNA or siRNA molecules according to one or several of the claims 1 through 11 consisting of
- at least one ampoule (ampoule A) containing a siRNA that is bonded, for the purpose of deactivation, to a selected peptide with a special amino acid sequence (for example the amino acid sequence -Pro-Leu-Ala-Leu-Trp-Ala-Arg- for the target cell enzyme matrix metalloproteinase-1) and/or at least one ampoule (ampoule B) that contains a PNA that is bonded, for the purpose of deactivation, to a selected peptide with a special amino acid sequence (for example the amino acid sequence -Pro-Leu-Ala-Leu-Trp-Ala-Arg- for the target cell enzyme matrix metalloproteinase-1)
- at least one further ampoule (ampule C) with a transfection system, for example nanoparticles or lipids,
- dilution and reaction buffers for the contents of the ampoules A to C,
- one or several probes or syringes with hollow needle and other materials required for the injection of the mixture of the contents of the ampoules into the medium that contains the target cells, and
- an instruction manual for the administration containing a list of the selectable peptide amino sequences for the corresponding enzyme of the target cell.

13. Applikationskit gemäß Anspruch 12, enthaltend wenigstens eine Substanz, beispielsweise Antikörper zur semi-selektiven Bindung der PNA- oder siRNA- Molekiile an Zielzellen.

14. Application kit according to claim 12, containing at least one substance, for example polyethylene glycol chains for the fixation of the peptide and the PNA or siRNA molecules.

15. Application kit according to claim 12, containing at least one substance, for example lipid-based transfection reagent, for the transfection of the PNA or siRNA molecules into the target cell.

16. Application kit according to claim 12, wherein it is prepared for in vitro or in vino applications.

## Revendications

1. Des molécules PNA ou siRNA pour la transfection biologiquement inactive dans une cellule cible afin d'y empêcher l'expression de gènes après son activation biologique par la liaison aux mRNA et, par exemple, dans le cas de siRNA sous la formation d'un complexe «RISC», sont **caractérisées en ce que** les molécules PNA ou siRNA (1) possèdent - aux fins de leur inactivation biologique à l'extérieur de la cellule cible - au moins une liaison covalente (8, 8'), qui peut seulement être fracturée par la ou les enzymes (9, 10) à spécificité cellulaire de cette cellule cible, avec un peptide (2, 3) au moins, qui présente respectivement une ou plusieurs séquences d'acide amino (11) pour la ou les enzymes choisies typiques aux cellules cibles et respectivement significatives pour la liaison covalente (8, 8') à fracturer.

2. Des molécules PNA ou siRNA suivant la revendication 1 sont **caractérisées en ce qu'**au moins un peptide (2, 3) - aux fins de fracturer la liaison (8, 8') par l'enzyme à spécificité cellulaire caspase-4 - possède la séquence d'acide amino -L-E-V-D- ou matrix métalloprotéinase-1, la séquence d'acide amino -Pro-Leu-Ala-Leu-Trp-Ala-Arg- ou matrix métalloprotéinases-2,7, la séquence d'acide amino -Pro-Leu-Gly-Leu-Dpa-Ala-Arg- ou matrix métalloprotéinases-2,9, la séquence d'acide amino -Pro-Leu-Gly-Met-Trp-Ser-Arg- ou matrix métalloprotéinases-3,1,2,9, la séquence d'acide amino -Arg-Pro-Lys-Pro-Tyr-Ala-Nval-Trp-Met-Lys- ou cathepsin-S, la séquence d'acide amino -Phe-Arg-Phe(p-nitro)- ou cathepsin-G, la séquence d'acide amino -Ala-Ala-Phe- ou cathepsin-D, la séquence d'acide amino -Arg-Gly-Phe-Phe-Leu- ou enzyme de conversion de l'angiotensine la séquence d'acide amino -Gly-His-Leu- ou enzyme de conversion de l'angiotensine, la séquence d'acide amino -Phe-Gly-Gly- ou enzyme de conversion de l'angiotensine, la séquence d'acide amino -Gly-p-Nitro-Phe-Pro-.

3. Le procédé permettant l'activation à spécificité cellulaire des molécules PNA ou siRNA dans ou à une cellule cible, pendant laquelle les molécules PNA ou siRNA sont transfectées de préférence de façon biologiquement inactive, afin d'y empêcher l'expression de gènes après son activation biologique par la liaison aux mRNA et, par exemple, dans le cas de siRNA sous la formation d'un complexe «RISC», est **caractérisée en ce que** le molécule PNA ou siRNA - aux fins de son inactivation biologique - à l'extérieur de la cellule cible et/ou avant la transfection est liée de façon covalente à un ou plusieurs peptides respectivement par au moins une séquence d'acide amino choisie pour la ou les enzymes typiques aux cellules cibles et significative pour la liaison covalente à fracturer et que la liaison covalente du ou des peptides à la molécule PNA ou siRNA est fracturée - aux fins de son activation biologique - par l'enzyme typique à la cellule cible.

4. Le procédé suivant la revendication 3 est **caractérisé en ce que** la molécule de PNA ou siRNA avec la ou les peptides liées de façon covalente pour améliorer la transfection dans les cellules cibles est couplée à un système de transfection.

5. Le procédé suivant la revendication 4 est **caractérisé en ce qu'**un système porteur, par exemple des nanoparticules avec ou sans marquage de ligand/anticorps/antigène, est utilisé comme système de transfection.

6. Le procédé suivant la revendication 4 est **caractérisé en ce que** des molécules, par exemple des nanoparticules ou des lipides avec lesquels les molécules de PNA ou siRNA étant sélectivement activables sont enveloppées pour réaliser leur transfection, servent de système de transfection.

7. Le procédé suivant la revendication 4 est **caractérisé en ce que** des méthodes à base de lipides sont utilisées comme système de transfection.

8. Le procédé suivant la revendication 4 est **caractérisé en ce qu'**un accompagnement par protéines TAT est prévu comme système de transfection.

9. Le procédé suivant la revendication 3 est **caractérisé en ce que** la molécule de PNA ou siRNA est transfectée en état inactive dans la cellule cible et est activée ici par la ou les enzymes à spécificité cellulaire.

10. Le procédé suivant la revendication 3 est **caractérisé en ce que** la molécule PNA ou siRNA inactivée par la liaison de peptide est déjà activée - directement avant la transfection dans la cellule cible - partiellement ou complètement (se référant au nombre total des liaisons covalentes des peptides) par une ou plusieurs enzymes à spécificité cellulaire existant sur leur surface ou dans leur environnement (p.ex. matrix métalloprotéinase-1, matrix métalloprotéinases-2, matrix métalloprotéinases-3, matrix métalloprotéinases-7, matrix métalloprotéinases-9).

11. Le procédé suivant la revendication 3 est **caractérisé en ce que** la molécule de PNA ou siRNA avec la ou les peptides liées de façon covalente est couplée à une ou plusieurs substances marqueurs.

12. Le kit d'application pour l'administration des molécules PNA ou siRNA suivant une ou plusieurs des revendications 1 à 11 se composant du moins
- au moins d'une ampoule (ampoule A), qui contient aux fins de l'inactivation une siRNA liée à une peptide sélectionnée avec une séquence spéciale d'acide amino (par exemple la séquence spéciale d'acide amino -Pro-Leu-Ala-Leu-Trp-Ala-Arg- pour l'enzyme des cellules cibles matrix métalloprotéinase-1) et/ou au moins d'une ampoule (ampoule B), qui contient aux fins de l'inactivation une PNA liée à une peptide sélectionnée avec une séquence spéciale d'acide amino (par exemple la séquence spéciale d'acide amino -Pro-Leu-Ala-Leu-Trp-Ala-Arg- pour l'enzyme des cellules cibles matrix métalloprotéinase-1),
- au moins d'une autre ampoule (ampoule C) avec un système de transfection, pas exemple des nanoparticules ou lipides,
- d'un tampon de dilution et de réaction pour les contenus des ampoules A à C,
- d'une ou plusieurs sondes et/ou seringues avec canule et d'autres matériaux nécessaires pour injecter le mélange formé des contenus des ampoules dans le milieu contenant les cellules cibles ainsi que
- des instructions pour l'administration avec un état comparatif des séquences de peptides amino à sélectionner pour les enzymes respectives de la cellule cible.

13. Le kit d'application suivant la revendication 12 contenant au moins une substance, par exemple des anticorps permettant la liaison semi sélective des molécules de PNA ou siRNA aux cellules cibles.

14. Le kit d'application suivant la revendication 12 contenant au moins une substance, par exemple des chaînes de polyéthylène glycol permettant l'ancrage de la peptide et des molécules de PNA ou siRNA.

15. Le kit d'application suivant la revendication 12 contenant au moins une substance, par exemple un réactif de transfection à base de lipides, pour la transfection des molécules de PNA ou siRNA dans la cellule cible.

16. Le kit d'application suivant la revendication 12 est **caractérisé en ce que** celui-ci est mis à disposition pour des applications in vitro ou vivo.
